# EUROPEAN PATENT APPLICATION

(11) **EP 4 652 960 A1**
(43) Date of publication of application: **26.11.2025**
(21) Application number: 24744129.8
(22) Date of filing: 09.01.2024
(51) Int. Cl.: A61C 7/00, A61C 7/08

(54) **METHOD AND APPARATUS FOR GENERATING DIGITAL MODEL OF CORRECTIVE DEVICE, AND MANUFACTURING METHOD FOR CORRECTIVE DEVICE**

(30) Priority: 19.01.2023 CN 202310074437
(71) Applicant: Air Force Medical University of PLA, Xi'an, Shaanxi 710032 (CN); BJ Appliance Health Science & Technology Co., Ltd., Beijing 100085 (CN); Elkem Silicones Shanghai Co., Ltd., Shanghai 201108 (CN)
(72) Inventor: WU, Junjie, Shaanxi 710032 (CN); WANG, Qingyu, Beijing 100085 (CN); JIA, Liya, Shanghai 201108 (CN); MA, Xiaokai, Beijing 100085 (CN); CHEN, Xia, Beijing 100085 (CN); HOU, Yafei, Beijing 100085 (CN); HAN, Haiwei, Beijing 100085 (CN)
(74) Representative: Meyer-Dulheuer MD Legal Patentanwälte PartG mbB
(86) International application number: PCT/CN2024/071454
(87) International publication number: WO 2024/152953

(57) **Abstract**

A method for generating a digital model of an orthodontic appliance, which comprises: determining overall size information and alveolar size information for a digital model of an orthodontic appliance according to information related to different dentition areas of a target oral cavity digital model; and generating the digital model of the orthodontic appliance according to the overall size information and the alveolar size information. Further provided are an apparatus for generating a digital model of an orthodontic appliance using the method for generating a digital model of an orthodontic appliance, as well as an orthodontic appliance manufactured using the method for generating a digital model of an orthodontic appliance. The method for generating a digital model of an orthodontic appliance can improve production efficiency and product precision of orthodontic appliances.

## Description

This application claims the priority of a Chinese patent application submitted to the Chinese Patent Office on 19 January 2023, with the application number 202310074437.9, and the invention name is "Method for generating digital model of orthodontic appliance, device and manufacturing method of orthodontic appliance". The entire content of this Chinese patent application is incorporated herein by reference.

### TECHNICAL FIELD

The present disclosure relates to the field of orthodontic appliance technology, particularly to a method for generating a digital model of an orthodontic appliance, a device for generating a digital model of an orthodontic appliance, a manufacturing method of an orthodontic appliance, a manufacturing device of an orthodontic appliance, an orthodontic appliance, an electronic device and a non-volatile computer-readable storage medium.

### BACKGROUND

Silicone-based orthodontic appliances are widely used in the treatment of malocclusion in children, and they have excellent effects in correcting bad oral habits, inducing tooth eruption, and establishing normal occlusion.

In related technologies, silicone-based orthodontic appliances are divided into two categories: pre-made and personalized customization.

Pre-made silicone orthodontic appliances are a type of mass-produced products. The product series of pre-made silicone orthodontic appliances are divided into multiple sizes and models. In clinical applications, the appropriate size model products suitable for the patient's oral condition are selected.

The personalized customized silicone orthodontic appliance is produced by collecting the patient's dental and facial data, combining the patient's specific conditions, and manually creating a mold. Compared with the pre-made silicone orthodontic appliance, the personalized customized silicone orthodontic appliance fits the patient's dental and facial features better, and has better applicability and comfort.

### SUMMARY

The inventors of this disclosure have discovered that the aforementioned related technologies have the following problems: the production process relies heavily on manual operations, resulting in a decrease in the production efficiency and product accuracy of the orthodontic appliances.

In view of this, this disclosure proposes a technical solution for generating a digital model of an orthodontic appliance, which can improve the production efficiency and product accuracy of the orthodontic appliance.

According to some embodiments of this disclosure, a method for generating a digital model of an orthodontic appliance is provided, which includes: determining the overall size information and the size information of the dental arches of the digital model of the target oral based on the relevant information of different tooth row areas; generating the digital model of the orthodontic appliance based on the overall size information and the size information of the dental arches.

In some embodiments, determining the overall size information of the orthodontic appliance digital model includes: determining the overall size information based on the widths of teeth in different dental regions, the widths between teeth, and the positions of the teeth.

In some embodiments, determining the overall size information includes: determining the overall width information of the orthodontic appliance digital model based on the widths of incisors in different dental regions, the widths between canines, the widths between premolars, and the widths between molars; determining the overall length information of the orthodontic appliance digital model based on the positions of the molars in different dental regions.

In some embodiments, the size information of the dental arches in the digital model of the orthodontic appliance includes: determining the size information of the dental arches based on the labial-buccal and lingual contours of different dental regions.

In some embodiments, determining the size information of the dental arches includes: smoothing the morphological data of the labial-buccal and lingual contours, and determining the smoothed morphological data; based on the smoothed morphological data, determining the size information of the dental arches.

In some embodiments, the generation method also includes: determining the height of the occlusal splint of the digital model of the orthodontic appliance based on the distance between the upper and lower occlusal surfaces of different dental regions, and the height of the occlusal splint is used to generate the digital model of the orthodontic appliance.

In some embodiments, determining the height of the jaw pad of the orthodontic appliance digital model includes: when the opening degree of the maxilla and mandible in the target oral digital model is less than the opening threshold, determining the distance between the upper occlusal surface and the lower occlusal surface.

In some embodiments, determining the height of the jaw pad of the orthodontic appliance digital model includes: determining the height of the digital model of the orthodontic appliance based on the resting occlusal gap of different dental regions.

In some embodiments, the generation method also includes: determining the length of the outer wall of the digital model of the orthodontic appliance based on the gingival position of the teeth in different dental regions, and the length of the outer wall is used to generate the digital model of the orthodontic appliance.

In some embodiments, determining the outer wall length of the orthodontic appliance digital model involves: smoothing the lines connecting the gum positions of different teeth in different dental regions; and determining the outer wall length based on the result of the smoothing process.

In some embodiments, the generation method also includes: adjusting the patient's initial oral digital model according to the target oral parameters to determine the target oral digital model.

In some embodiments, adjusting the patient's initial oral digital model comprises: using the initial mandibular and maxillary digital models in the initial oral digital model to fit a preset shape of the dental arch, thereby determining the target mandibular and maxillary digital models in the target oral digital model.

In some embodiments, adjusting the patient's initial oral digital model comprises: based on preset overbite parameters, maxillary and mandibular dental arch parameters, overjet parameters, incisor relationship, molar relationship, or the Spee curve among at least one of these, adjusting the initial occlusal relationship in the initial oral digital model to determine the target occlusal relationship in the target oral digital model.

In some embodiments, the initial oral digital model includes the initial upper and lower jaw digital models, and the initial oral digital model also includes at least one of the initial occlusal relationship or oral soft tissue information. The initial upper and lower jaw digital models are obtained through an intraoral scanner or the patient's dental impression, the initial occlusal relationship is obtained through an intraoral scanner or a jaw frame, and the oral soft tissue information is obtained through optical scanning technology, X-ray imaging technology, ultrasound imaging technology, CT (Computed Tomography, computer tomography) scanning or nuclear magnetic resonance technology.

In some embodiments, the dental arch area includes multiple areas such as the incisor area, the canine area, the premolar area or the molar area.

In some other embodiments of the present disclosure, a device for generating a digital model of an orthodontic appliance is provided, which comprises: a determination unit, which is used to determine the overall size information and the size information of the dental arches of the orthodontic appliance digital model based on the relevant information of different tooth row areas of the target oral digital model; a generation unit, which is used to generate the orthodontic appliance digital model based on the overall size information and the size information of the dental arches.

In some embodiments, the determination unit determines the overall size information based on the width of teeth, the width between teeth, and the position of teeth in different tooth row areas.

In some embodiments, the determination unit determines the overall width information of the orthodontic appliance digital model based on the widths of the incisors in different dental arch regions, the widths between the canines, the widths between the premolars, and the widths between the molars; and determines the overall length information of the orthodontic appliance digital model based on the positions of the molars in different dental arch regions.

In some embodiments, the determination unit determines the size information of the dental arch based on the labial-buccal and lingual contours of different dental arch regions.

In some embodiments, the determination unit smooths the morphological data of the labial-buccal and lingual contours, determines the smoothed morphological data; and determines the size information of the dental arch based on the smoothed morphological data.

In some embodiments, the determination unit determines the height of the jaw pad of the orthodontic model based on the distance between the upper and lower occlusal surfaces of different dental arch regions, and the height of the jaw pad is used to generate the orthodontic model.

In some embodiments, the determination unit determines the distance between the upper and lower occlusal surfaces when the opening degree of the upper and lower jaws in the target oral digital model is less than the opening threshold.

In some embodiments, the determination unit determines the height of the jaw pad of the orthodontic model based on the resting interarch gap of different dental arch regions.

In some embodiments, the determination unit determines the outer wall length of the digital model of the orthodontic appliance based on the gingival positions of the teeth in different dental regions, and the outer wall length is used to generate the digital model of the orthodontic appliance.

In some embodiments, the determination unit smooths the connections of the gingival positions of different teeth in different dental regions; and based on the result of the smoothing processing, determines the outer wall length.

In some embodiments, the generating device further includes: an adjustment unit, which is used to adjust the patient's initial oral digital model based on the target oral parameters to determine the target oral digital model.

In some embodiments, the adjustment unit uses the initial oral digital model's initial upper and lower jaw digital models to fit the pre-set shape of the dental arch form to determine the target upper and lower jaw digital models in the target oral digital model.

In some embodiments, the adjustment unit adjusts the initial occlusal relationship in the initial oral digital model based on the preset occlusal parameters, maxillary and mandibular dental arch parameters, covering parameters, incisor relationship, molar relationship, or at least one of the Spee curve, in order to determine the target occlusal relationship in the target oral digital model.

In some embodiments, the initial oral digital model includes the initial upper and lower jaw digital models. The initial oral digital model includes at least one of the initial occlusal relationship or oral soft tissue information. The initial upper and lower jaw digital models are obtained through intraoral scanners or the patient's dental impressions. The initial occlusal relationship is obtained through intraoral scanners or jaw frames. The oral soft tissue information is obtained through optical scanning technology, X-ray imaging technology, ultrasound imaging technology, CT scanning or nuclear magnetic resonance technology.

In some embodiments, the dental region comprises multiple areas such as the incisor area, the canine area, the premolar area or the molar area.

According to other some embodiments of the present disclosure, a manufacturing method for an orthodontic appliance is provided, which comprises: using 3D printing technology to manufacture the orthodontic appliance based on theorthodontic appliance digital model generated according to the generation method of the orthodontic appliance digital model.

In some embodiments, use the first extrusion head to extrude the main material of the orthodontic appliance, and use the second extrusion head to extrude the supporting material.

In some embodiments, the first extrusion head and the second extrusion head can avoid each other during the printing process in accordance with the instructions.

In some embodiments, the main material of the orthodontic appliance is printed using the first extrusion head, and the supporting material of the orthodontic appliance is printed using the second extrusion head. This involves: after the first extrusion head and the second extrusion head have completed the extrusion and stacking molding process for all layers of the orthodontic appliance, the printed piece is placed at room temperature or under heating conditions to allow all the main material layers to solidify. The curing temperature ranges from room temperature to 180°C, and preferably involves heating for curing, such as at 80°C. Although the main material and the supporting material are heated and cured together, the supporting material is a non-reactive product and will not solidify when heated.

In some embodiments, the main material is made from a curable silicone rubber formulation system.

In some embodiments, the thermosetting silicone rubber formulation contains vinyl silicone oil, hydrogen-containing silicone oil, platinum-based catalyst, and reinforcing agent.

In some embodiments, the reinforcing agent comprises silica.

In some embodiments, the thermosetting silicone rubber formulation contains components such as thixotropic agents.

In some embodiments, the hardness range of the main material is 40 to 80 Shore A.

In some embodiments, the hardness range of the main material is 50 to 70 Shore A.

In some embodiments, the hardness range of the main material is 55 to 65 Shore A.

In some embodiments, the tensile strength of the main material is greater than 3 MPa.

In some embodiments, the tensile strength of the main material is greater than 5 MPa.

In some embodiments, the elongation at break of the main material is greater than 70%.

In some embodiments, the elongation at break of the main material is greater than 100%.

In some embodiments, the tear strength of the main material is greater than 9 N/mm.

In some embodiments, the tear strength of the main material is greater than 10 N/mm.

In some embodiments, the diameter of the main material needle is 0.2 to 0.8 mm, the diameter of the main material needle is preferably 0.4 mm, the diameter of the supporting material needle is 0.2 to 0.8 mm, and the diameter of the supporting material needle is preferably 0.4 mm.

In some embodiments, the extrusion width of the main material is 0.2 to 0.8 mm, the extrusion width of the supporting material is 0.2 to 0.8 mm, the filling rate of the main material is 85 to 100%, the filling rate of the supporting material is 70 to 100%, the printing speed of the orthodontic appliance is 10 to 80 mm/s, the layer thickness is 0.2 to 0.3 mm.

In some embodiments, the diameter of the main material needle is preferably 0.4 mm, the diameter of the supporting material needle is preferably 0.4 mm.

In some embodiments, the extrusion width of the main material is preferably 0.4 mm, the extrusion width of the supporting material is preferably 0.4 mm, the filling rate of the main material is preferably 90%, the filling rate of the supporting material is preferably 90 to 100%, the printing speed of the orthodontic appliance is preferably 25 mm/s, and the layer thickness is preferably 0.2 mm, the supply rate of the main materials and supporting materials is 80%.

According to other some embodiments of the present disclosure, an electronic device is provided, which comprising: Memory; and The processor coupled to the memory, the processor being configured to execute the method for generating the digital model of the said orthodontic appliance based on the instructions stored in the memory.

According to another some embodiments of the present disclosure, a non-volatile computer-readable storage mediumis provided, on which a computer program is stored. When executed by a processor, this program realizes the generation method of the said orthodontic digital model.

In the above-mentioned embodiment, based on the specific conditions of different tooth arrangement areas desired by the patient, a suitable orthodontic appliance is generated. Thus, by utilizing computer technology, personalized and accurate orthodontic appliance designs can be automatically realized for the patient, thereby enhancing the production efficiency and product accuracy of the orthodontic appliances.

### BRIEF DESCRIPTION OF THE DRAWINGS

The attached figures form part of the specification and describe the embodiments of the present disclosure, and together with the specification, are used to explain the principles of the present disclosure.

Referring to the figures, according to the following detailed description, the present disclosure can be more clearly understood:
Figure 1 shows a flowchart of some embodiments of the method for generating the digital model of the orthodontic appliance;
Figure 2 shows a flowchart of some other embodiments of the method for generating the digital model of the orthodontic appliance;
Figure 3 shows a block diagram of some embodiments of the device for generating the digital model of the orthodontic appliance;
Figure 4 shows a block diagram of some embodiments of the electronic device;
Figure 5 shows a block diagram of some other embodiments of the electronic device.

### DETAILED DESCRIPTION OF THE EMBODIMENTS

Now, referring to the attached drawings, various exemplary embodiments of the present disclosure will be described in detail. It should be noted that: unless otherwise specifically stated, the relative arrangement, numerical expressions and numerical values of the components and steps described in these embodiments do not limit the scope of the present disclosure.

At the same time, it should be understood that, for the convenience of description, the sizes of the various parts shown in the attached drawings are not drawn in accordance with the actual proportion.

The description of at least one exemplary embodiment herein is merely illustrative and does not limit the present disclosure, its application or use in any way.

Technologies, methods and devices known to those skilled in the relevant field may not be discussed in detail, but in appropriate cases, the technologies, methods and devices should be regarded as part of the specification.

In all the examples shown and discussed herein, any specific values should be interpreted merely as exemplary and not as limitations. Therefore, other exemplary embodiments may have different values.

It should be noted that similar numerals and letters in the following drawings represent similar items, and once an item is defined in one drawing, it does not need to be further discussed in the subsequent drawings.

As mentioned earlier, on one hand, oral problems (especially those in children) and dental-jaw-facial growth conditions have individualized characteristics, which leads to deficiencies in terms of accuracy and applicability in the clinical application of pre-made silicone orthodontic appliances; on the other hand, the production process of personalized silicone orthodontic appliances is highly dependent on manual labor, and the production efficiency and quality are greatly influenced by the level of manual work.

In response to the aforementioned technical issues, this disclosure constructs a complete set of digital and intelligent design system for personalized customized silicone orthodontic appliances, a 3D printing preparation system, and corresponding products, enabling the digital, intelligent and automated production and application of personalized customized silicone orthodontic appliances, reducing reliance on manual labor and improving product accuracy.

For example, the technical solution of the present disclosure can be achieved through the following implementation examples.

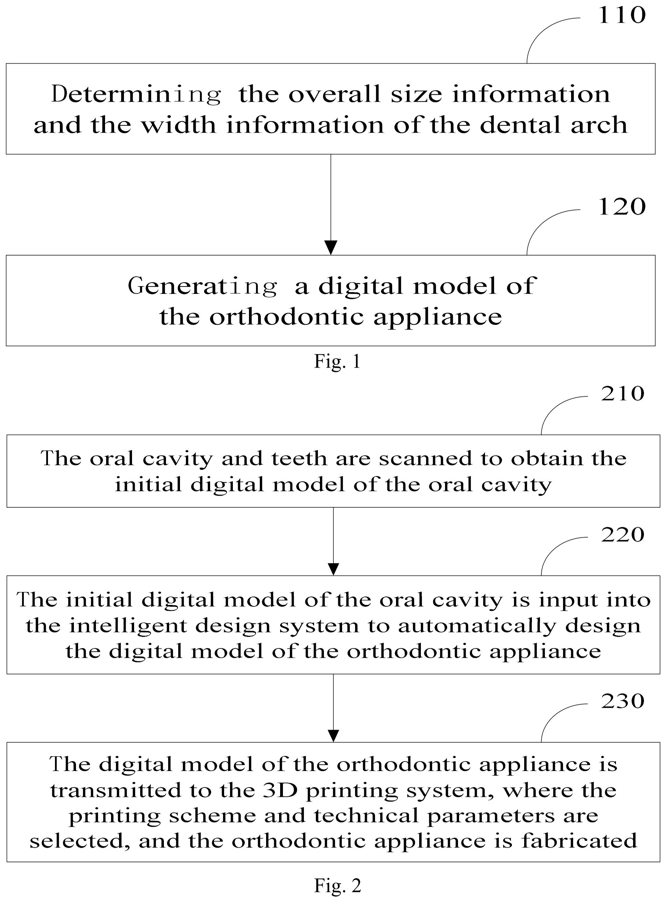

Figure 1 shows the flowchart of some implementation examples of the method for generating the digital model of the orthodontic appliance in the present disclosure.

As shown in Figure 1, in step 110, the overall size information and the size information of the dental arch of the digital model of the orthodontic appliance are determined based on the relevant information of different tooth row areas of the target oral digital model. For example, the size information of the dental arch includes at least one of the width or length of the dental arch.

In some embodiments, the initial oral digital model of the patient is adjusted based on the target oral parameters to determine the target oral digital model.

In some embodiments, the initial oral digital model includes the initial upper and lower jaw digital models. The initial oral digital model includes at least one of the initial occlusal relationship or oral soft tissue information. The initial upper and lower jaw digital models are obtained through intraoral scanners or the patient's dental impressions. The initial occlusal relationship is obtained through intraoral scanners or jaw frames. The oral soft tissue information is obtained through optical scanning technology, X-ray imaging technology, ultrasound imaging technology, CT scanning or nuclear magnetic resonance technology.

For example, the initial digital models of the upper and lower jaws, the occlusal relationship of the teeth, and the information of the oral soft tissues need to be obtained first.

For example, the digital models of the upper and lower jaws can be obtained in the following two ways: by using an intraoral scanner to obtain the digital models of the upper and lower jaws with the initial occlusal relationship; by having the patient perform occlusion and obtaining dental impressions with alginate or other impression materials, and then using gypsum or other materials to make dental gypsum models, and finally using laser to scan the gypsum models to generate the digital models of the upper and lower jaws.

For example, the initial occlusal relationship of teeth can be obtained in the following ways: dental scanners, jaw frames, etc.

For example, oral soft tissues can include at least one of the upper lip, lower lip, upper cheek, lower cheek, upper jaw skin, lower jaw skin, tongue, and frenulum. Information about oral soft tissues can be obtained through the following methods: optical scanning technology, X-ray or ultrasound imaging technology, CT scanning or nuclear magnetic resonance technology, etc., to obtain the digital model of oral soft tissues.

In some embodiments, the computer software can automatically adjust the initial upper and lower jaw digital models based on parameters such as the dental arch shape, overjet parameters, overbite parameters, or molar relationship, in order to obtain the target upper and lower jaw digital models and the target occlusal relationship. Additionally, the obtained target upper and lower jaw digital models and the target occlusal relationship can be subject to manual review and adjustment.

In some embodiments, the initial maxillary and mandibular digital models in the initial oral digital model are utilized to fit the pre-defined shape of the dental arch, thereby determining the target maxillary and mandibular digital models in the target oral digital model.

For example, the standard "egg-shaped" dental arch shape is fitted in the dental arch area (such as incisors, canines, molars, etc.) to determine the target oral digital model.

In some embodiments, based on the preset occlusal parameters, maxillary and mandibular dental arch parameters, overjet parameters, incisor relationship, molar relationship or at least one of the Spee curve, the initial occlusal relationship in the initial oral digital model is adjusted to determine the target occlusal relationship in the target maxillary and mandibular digital models.

For example, based on standard occlusal parameters, standard coverage parameters, neutral molar relationship (such as normal mesiodistal positions of the maxilla and mandible, as well as the upper and lower dental arches, and a neutral molar relationship, when the centric occlusion position is reached, the mesiobuccal cusp of the first permanent maxillary molar contacts the mesiobuccal groove of the first permanent mandibular molar), the initial occlusal relationship is adjusted to determine the target occlusal relationship.

In some embodiments, overall size information is determined based on the width of teeth in different dental regions, the width between teeth, and the position of the teeth. For example, the dental regions include multiple areas such as the incisor region, the canine region, the premolar region, or the molar region.

For instance, computer software, based on the target oral digital models with target occlusal relationships, automatically fits the pre-set digital models of orthodontic appliances to generate the digital models of the orthodontic appliances.

In some embodiments, the overall size (such as width and length) of the digital model of the orthodontic appliance is adjusted according to different dental regions (such as incisors, canines, molar regions, etc.).

For example, the fitting width can be calculated based on the width of the incisors, the width between the canines, and the width between the first molars. The fitting length can be calculated based on the position of the second molars, etc.

In some embodiments, the overall width information of the orthodontic appliance digital model is determined based on the widths of the incisors in different dental regions, the widths between the canines, the widths between the premolars, and the widths between the molars; and the overall length information of the orthodontic appliance digital model is determined based on the positions of the molars in different dental regions.

In some embodiments, the size information of the dental arches is determined based on the labial-buccal and lingual contours of different dental arch regions.

For example, the morphological data of the labial-buccal and lingual contours are smoothed, and the smoothed morphological data are determined; based on the smoothed morphological data, the size information of the dental arches is determined.

For instance, the width of the dental arches in different dental arch regions (such as incisors, canines, molar regions, etc.) after smoothing the labial-buccal and lingual contours is fitted to the digital model of the orthodontic appliance.

In some embodiments, the height of the jaw pad of the digital model of the orthodontic appliance is determined based on the distance between the upper and lower occlusal surfaces of different dental arch regions. The height of the jaw pad is used to generate the digital model of the orthodontic appliance.

For example, when the opening degree of the upper and lower jaws in the target oral digital model is less than the opening threshold, the distance between the upper and lower occlusal surfaces is determined.

For example, the height of the jaw pad of the digital model of the orthodontic appliance is determined based on the resting interarch gap of different dental arch regions.

For instance, when the target oral digital model with a target occlusal relationship is in a slightly open state, the height of the jaw pad in the digital model of the orthodontic appliance is adjusted based on the distances of the upper and lower occlusal surfaces of different tooth regions (such as incisors, canines, molar regions, etc.) after smoothing processing.

For example, the height of the jaw pad in the digital model of the orthodontic appliance is adjusted according to the resting occlusal gap. The resting occlusal gap refers to the state formed between the upper and lower teeth when the mouth is not open, no speaking, no swallowing, the lower jaw is in a resting state, the upper and lower jaws are separated, the teeth are not in contact, and there is a wedge-shaped gap from the pharynx to the lips. This is the state where the upper and lower teeth regions are larger in the front and smaller at the back.

In some embodiments, the outer wall length of the digital model of the orthodontic appliance is determined based on the gingival position of the teeth in different dental regions. The outer wall length is used to generate the digital model of the orthodontic appliance.

In some embodiments, the connections of the gingival positions of different teeth in different dental regions are smoothed; based on the result of the smoothing processing, the outer wall length is determined.

For example, based on the line connecting the gum positions between two teeth in different dental regions (such as incisors, canines, molars, etc.) after smoothing treatment, the length of the outer wall of the digital model of the orthodontic appliance is adjusted. The length of the outer wall can ensure that the orthodontic appliance does not press on the gums, such as when the length of the outer wall of the orthodontic appliance is not at the gum position.

In step 120, a digital model of the orthodontic appliance is generated based on the overall size information and the size information of the tooth sockets.

In some embodiments, a digital model of the orthodontic appliance is generated based on the height of the jaw pad, the length of the outer wall, the overall size information and the size information of the tooth sockets.

For example, a technician can review the digital model of the orthodontic appliance automatically adjusted by the computer software; according to the treatment plan (such as treatment cycle, treatment sequence, etc.) and the condition of the dentition (such as missing teeth, abnormal teeth position, etc.), the digital model of the orthodontic appliance can be adjusted to meet the treatment needs of the patient.

Figure 2 illustrates the flowchart of some other embodiments of the method for generating the digital model of the orthodontic appliance disclosed herein.

As shown in Figure 2, in step 210, the oral cavity and teeth are scanned to obtain the initial digital model of the oral cavity.

In step 220, the initial digital model of the oral cavity is input into the intelligent design system to automatically design the digital model of the orthodontic appliance.

In step 230, the digital model of the orthodontic appliance is transmitted to the 3D printing system, where the printing scheme and technical parameters are selected, and the orthodontic appliance is fabricated.

In the above-mentioned implementation example, the application software is used to intelligently design the silicone orthodontic appliances. On one hand, this enhances the personalization and precision of the products, thereby improving the treatment outcome. On the other hand, it enables the automatic innovation of the orthodontic appliance design process, avoiding the technical problem of excessive reliance on human factors in the manual design process and improving the quality of the appliances.

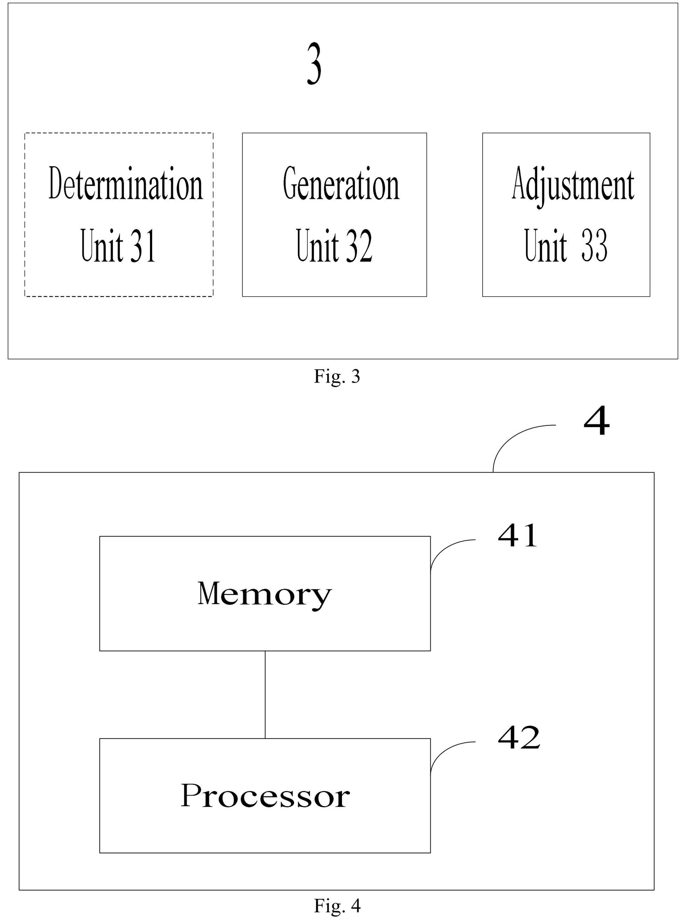

Figure 3 illustrates the block diagram of some embodiments of the device for generating the digital model of the orthodontic appliance of the present disclosure.

As shown in Figure 3, the device for generating the digital model of the orthodontic appliance 3 includes: a determination unit 31, which is used to determine the overall size information and the size information of the dental arch of the orthodontic appliance digital model based on the relevant information of different tooth row areas of the target oral digital model; a generation unit 32, which is used to generate the orthodontic appliance digital model based on the overall size information and the size information of the dental arch.

In some embodiments, the determination unit 31 determines the overall size information based on the width of teeth in different dental regions, the width between teeth, and the position of the teeth.

In some embodiments, the determination unit 31 determines the overall width information of the orthodontic appliance digital model based on the width of the incisors in different dental regions, the width between the canines, the width between the premolars, and the width between the molars; and determines the overall length information of the orthodontic appliance digital model based on the position of the molars in different dental regions.

In some embodiments, the determination unit 31 determines the size information of the dental arch based on the labial-buccal contour and the lingual contour of different dental regions.

In some embodiments, the determination unit 31 smooths the shape data of the labial-buccal contour and the lingual contour, and determines the smoothed shape data; based on the smoothed shape data, the size information of the dental arch is determined.

In some embodiments, the determination unit 31 determines the height of the occlusal splint of the digital model of the orthodontic appliance according to the distance between the upper and lower occlusal surfaces of different tooth row regions, and the height of the occlusal splint is used to generate the digital model of the orthodontic appliance.

In some embodiments, when the opening degree of the upper and lower jaws of the target oral digital model is less than the opening threshold, the determination unit 31 determines the distance between the upper occlusal surface and the lower occlusal surface.

In some embodiments, the determination unit 31 determines the height of the jaw pad of the orthodontic appliance digital model based on the resting intercuspal gap of different dental arch regions.

In some embodiments, the determination unit 31 determines the outer wall length of the orthodontic appliance digital model based on the gingival position of teeth in different dental arch regions. The outer wall length is used to generate the orthodontic appliance digital model.

In some embodiments, the determination unit 31 smooths the connection lines of the gingival positions of different teeth in different dental arch regions; based on the result of the smoothing processing, the outer wall length is determined.

In some embodiments, the generating device 3 further includes: an adjustment unit 33, which is used to adjust the patient's initial oral digital model based on the target oral parameters to determine the target oral digital model.

In some embodiments, the adjustment unit 33 uses the initial oral digital model's initial maxillary and mandibular digital models to fit the preset shape of the dental arch form to determine the target oral digital model's target maxillary and mandibular digital models.

In some embodiments, the adjustment unit 33 adjusts the initial oral digital model's initial occlusal relationship based on the preset overbite parameters, maxillary and mandibular dental arch parameters, overjet parameters, incisor relationship, molar relationship, or Spee curve (at least one of them) to determine the target oral digital model's target occlusal relationship.

In some embodiments, the initial oral digital model includes the initial maxillary and mandibular digital models. The initial oral digital model includes at least one of the initial occlusal relationship or oral soft tissue information. The initial maxillary and mandibular digital models are obtained through intraoral scanners or the patient's dental impressions. The initial occlusal relationship is obtained through intraoral scanners or jaw frames. The oral soft tissue information is obtained through optical scanning technology, X-ray imaging technology, ultrasound imaging technology, CT scanning or nuclear magnetic resonance technology.

In some embodiments, the dentition region comprises multiple areas such as the incisor area, the canine area, the premolar area, or the molar area.

In the above embodiments, by collecting the patient's dental and facial data and combining it with the treatment plan for the digital design and restoration of the orthodontic appliance, the digital model of the orthodontic appliance is directly used for 3D printing, thereby achieving digital and intelligent manufacturing.

In some embodiments, the manufacturing device of the orthodontic appliance can be a silicone 3D printing system, which includes at least two extrusion heads to print the main material and the supporting material respectively.

For example, the main material is made from a thermosetting silicone rubber formulation system, which contains vinyl silicone oil, hydrogen-containing silicone oil, platinum-based catalysts, reinforcing agents (such as silica aerogel, etc.). In addition, it also contains thixotropic agents and other components to ensure that the main material does not collapse during the printing process.

For example, the supporting material is used to temporarily support the main material when printing complex structural components, especially when printing suspended structures. After the printing is completed, when the main material has solidified, the supporting material can be removed by water or an appropriate solvent.

In some embodiments, the silicone 3D printing system includes forming methods such as printing while curing or post-curing.

For example, the features of the printing while curing system include the extrusion head of the printer extruding the main material while simultaneously undergoing curing through light or heat; or after printing one layer, curing through light or heat is performed, then another layer is printed and then cured through light or heat; this process is repeated until the printing piece is completed.

For example, the post-curing molding system enables the main material to remain unsolidified or to solidify slowly during the extrusion printing process; after each layer is extruded and stacked for molding, it can be placed in a high-temperature oven or exposed to UV radiation for curing all at once.

For instance, the suitable properties of the organic silicon main material can include a hardness range of 40 to 80 Shore A, preferably 50 to 70 Shore A, and more preferably 55 to 65 Shore A; a tensile strength greater than 3 MPa, preferably greater than 5 MPa; a tensile elongation greater than 70%, preferably greater than 100%; and a tear strength greater than 7 N/mm, preferably greater than 10 N/mm.

For example, in the case of the post-curing type system, the printer has a dual extrusion head printing function, which can respectively extrude the main material and the supporting material. The extrusion head has a Z-axis avoidance function, which can avoid interference and scratching problems during the dual extrusion head printing process. After the equipment is calibrated, it can be started for printing with just one click until the end, and the process can be unmanned. The main material can use AMSILSILIBIONE24503-65A/B, and the supporting material can use AMSILSILIBIONE92101.

In some embodiments, the main material and the supporting material are placed in the designated position of the printer; the silicone orthodontic model data is first converted to a format (such as the commonly used format of STL), and then the STL-format model is inspected and repaired.

In some embodiments, the diameter of the main material needle is 0.2 to 0.8 mm, and the preferred diameter of the main material needle is 0.4 mm. The diameter of the supporting material needle is 0.2 to 0.8 mm, and the preferred diameter of the supporting material needle is 0.4 mm.

In some embodiments, the extrusion width of the main material is 0.2 to 0.8 mm, and the extrusion width of the supporting material is 0.2 to 0.8 mm. The filling rate of the main material is 85 to 100%, and the filling rate of the supporting material is 70 to 100%. The printing speed of the orthodontic appliance is 10 to 30 mm/s, and the layer thickness is 0.2 to 0.3 mm. The preferred extrusion width of the main material is 0.4 mm, the preferred extrusion width of the supporting material is 0.4 mm, the preferred filling rate of the main material is 90%, and the preferred filling rate of the supporting material is 90 to 100%. The preferred printing speed of the orthodontic appliance is 25 mm/s, and the preferred layer thickness is 0.2 mm.

For example, the diameter of the main material needle is 0.4mm, the diameter of the supporting material needle is also 0.4mm. Other process parameters are set as follows: the extrusion width (main material) is 0.4mm, the extrusion width (support material) is 0.4mm, the filling rate (main material) is 90%, the filling rate (support material) is 70%, the printing speed is 25mm/s, the layer thickness is 0.2mm, the filling rate is 100%, and the material supply rate is 80%.

In some embodiments, the printed orthodontic appliance is placed in an 80°C oven for heating and curing for 30 minutes to 60 minutes, then heated to 120°C for another 15 minutes to complete the final curing and shaping of the product; the cured product is then removed, rinsed with water to remove the support, and dried.

For example, the relevant printing process parameters can also be as follows: Firstly, the silicone orthodontic model data is converted to a common format such as STL; then the STL model is inspected and repaired; the needle diameter of the main material is 0.4mm, and the needle diameter of the supporting material is also 0.4mm.
For example, other process parameter settings are as follows: Extrusion width (main material) 0.4mm, Extrusion width (support material) 0.4mm, Filling rate (main material) 90%, Filling rate (support material) 70%, Printing speed 25mm/s, Layer thickness 0.2mm, Filling rate 100%, Material supply rate 80%.

In some embodiments, the extrusion 3D printing system 41 includes a first extrusion head for printing the main material of the orthodontic appliance and a second extrusion head for printing the supporting material of the orthodontic appliance.

In some embodiments, after the first extrusion head completes the filling and scanning of the current layer of the orthodontic appliance, the control unit causes the second extrusion head to avoid and perform curing treatment on the current layer.

In some embodiments, the extrusion 3D printing system includes two molding methods: printing while curing or post-curing.

For example, the first extrusion head fills and scans the current layer of the orthodontic appliance according to the scanning path; the control of the second extrusion head avoids it; after the current layer is cured, the second extrusion head fills the next layer of the orthodontic appliance according to the scanning path; the first extrusion head and the second extrusion head repeat the above steps until all layers of the orthodontic appliance are printed.

For example, the on-the-fly printing and solidification method includes: the first extrusion head, based on the scanning path, completes the filling scan of each layer, then the second extrusion head system takes a detour; using a heat source or UV ultraviolet method (such as with wavelengths of 355nm, 405nm, etc.) for inter-layer heat radiation or UV irradiation for immediate solidification, the immediate solidification can be controlled by the temperature of the heat source or the intensity and duration of UV irradiation; after the solidification is completed, the platform descends a distance equal to one layer thickness, and the second extrusion head continues to fill according to the scanning path; the process of "scanning and filling + solidification" is repeated for each layer until the printing is completed.

For instance, after the first extrusion head and the second extrusion head have completed the extrusion and stacking molding process for all layers of the orthodontic appliance, the printed piece is cured to solidify all the main material layers.
For example, the post-curing molding method includes: through the design of the material formula, it is possible to not solidify or to solidify slowly (with a curing speed less than the threshold speed) during the extrusion process. After each layer is extruded and stacked for molding, it is then placed in a high-temperature oven or subjected to UV irradiation for curing at once.

In the above-mentioned embodiments, the silicone 3D printing systems all include two extrusion heads, which are used to print the main material and the supporting material respectively. The extrusion heads have a Z-axis avoidance function, which is suitable for the extrusion printing of liquid silicone materials. Thus, it can be applied to the printing of complex structural parts, especially those that require the addition of supporting elements for auxiliary molding, thereby improving production efficiency and quality.

In some embodiments, the digital model of the orthodontic appliance produced is first converted to a different format. For example, the converted format of the digital model of the orthodontic appliance is STL (Standard Template Library). The STL format digital model is then inspected and repaired.
For instance, a second extrusion head with a diameter of 0.4mm and appropriate process parameters can be selected for parametric slicing processing. The process parameters can include a scanning speed of 15mm/s, a layer thickness of 0.2mm, a filling rate of 100%, and a material supply rate of 80%, etc.

For example, the printing data of the slices of the orthodontic model digital model is transmitted to the 3D silicone printing system. The extrusion 3D printing system can undergo post-curing molding. It has a dual-head extrusion printing function and both are equipped with an automatic cutoff system, capable of printing the main body and auxiliary supporting materials.

For example, the main material can be a two-component liquid silicone with a Shore hardness of 65A; the supporting material can be soluble in water. The second extrusion head of the printer has a Z-axis avoidance function, which can avoid interference and scratching problems during the scanning jump process of the dual nozzles. After equipment calibration, the printing can be started with one click until completion, and the process can be unmanned.
For example, place the printed orthodontic appliance in an 80°C oven, heat and cure for 30 minutes to 60 minutes, then raise the temperature to 120°C for another 15 minutes to complete the final curing and shaping of the product.

For example, the solidified products are taken out for removing support water washing and drying.

In the above-mentioned embodiment, 3D printing software and equipment are used to produce silicone orthodontic appliances, applying digital technology in the production process. Compared with the manual pouring and grinding production technology of silicone orthodontic appliances, the production technology disclosed in this application has been innovated, achieving automated production, avoiding the influence of human operation errors, and improving the production process level and product quality.

Moreover, this application can reduce the constraints of production sites, improve the production mode of orthodontic appliances, and contribute to the improvement of treatment levels.

Figure 4 illustrates the block diagram of some embodiments of the electronic device of the present disclosure.

As shown in Figure 4, the embodiment of the electronic device 4 includes: a memory 41 and a processor 42 coupled to the memory 41. The processor 42 is configured to execute the generation method of the orthosis digital model in any embodiment of the present disclosure based on the instructions stored in the memory 41.

Here, the memory 41, for example, can include system memory, fixed non-volatile storage media, etc. The system memory, for example, stores an operating system, applications, boot loader Boot Loader, database, and other programs, etc.

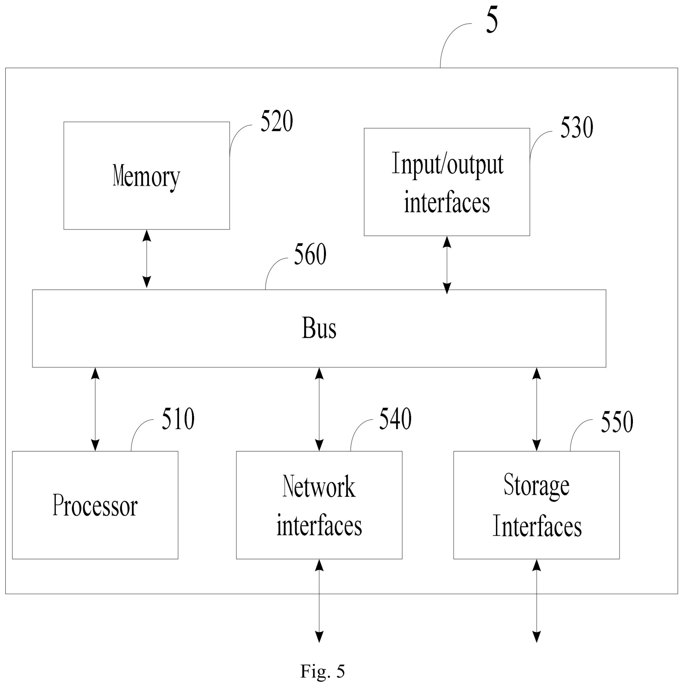

Figure 5 shows the block diagram of some other embodiments of the electronic device of the present disclosure.

As shown in Figure 5, the device 5 of this embodiment includes: a memory 510 and a processor 520 coupled to the memory 510. The processor 520 is configured to execute the generation method of the corrective digital model in any of the aforementioned embodiments based on the instructions stored in the memory 510.

Memory 510, for example, can include system memory, fixed non-volatile storage media, etc. The system memory, for instance, stores an operating system, applications, boot loader, and other programs, etc.

The electronic device 5 can also include input/output interfaces 530, network interfaces 540, storage interfaces 550, etc. These interfaces 530, 540, 550, as well as memory 510 and processor 520, are typically connected to each other through a bus 560. Among them, the input/output interface 530 provides connection interfaces for input/output devices such as displays, mice, keyboards, touch screens, microphones, speakers, etc. The network interface 540 provides connection interfaces for various networked devices. The storage interface 550 provides connection interfaces for external storage devices such as SD cards, USB drives, etc.

Those skilled in the art should understand that the embodiments of the present disclosure can be provided as methods, systems, or computer program products. Therefore, the present disclosure can take the form of a fully hardware implementation, a fully software implementation, or an implementation combining software and hardware aspects. Moreover, the present disclosure can be implemented in the form of a computer program product that is stored on one or more computer-readable non-transient storage media, including but not limited to disk storage, CD-ROM, optical storage, etc.

At this point, the generation method of the digital model of the orthodontic appliance, the generation device of the digital model of the orthodontic appliance, the manufacturing method of the orthodontic appliance, the manufacturing device of the orthodontic appliance, the orthodontic appliance, the electronic device and the non-volatile computer-readable storage medium according to this disclosure have been described in detail. To avoid obscuring the conception of this disclosure, some details that are commonly known in the field have not been described. Based on the above description, those skilled in the art can fully understand how to implement the technical solutions disclosed herein.

The method and system disclosed herein can be realized in many ways. For example, they can be implemented through software, hardware, firmware, or any combination of software, hardware, and firmware. The above-mentioned sequence of steps for the method is only for illustration purposes. The steps of the disclosed method are not limited to the specific sequence described above, unless otherwise specifically stated. Moreover, in some embodiments, the disclosed method can also be implemented as a program recorded on a recording medium, and these programs include machine-readable instructions for implementing the method according to the present disclosure. Therefore, the present disclosure also covers the recording medium for storing the program for executing the method according to the present disclosure.

Although some specific embodiments of the present disclosure have been elaborately described through examples, those skilled in the art should understand that the above examples are merely for illustration and do not limit the scope of the present disclosure. The skilled in the art should also understand that the above embodiments can be modified without departing from the scope and spirit of the present disclosure. The scope of the present disclosure is defined by the appended claims.

## Claims

1. A method for generating a digital model of an orthodontic appliance comprises:
Based on the relevant information of different tooth row areas of the target oral digital model, determine the overall size information and the size information of the dental arches of the orthodontic appliance digital model;
Based on the overall size information and the size information of the tooth sockets, generate the orthodontic appliance digital model.

2. The method according to claim 1, wherein the determination of the overall size information of the orthodontic appliance digital model includes:
Determining the overall size information based on the widths of teeth in different dental regions, the widths between teeth, and the positions of the teeth.

3. The method according to claim 2, wherein the determination of the overall size information includes:
Determining the overall width information of the orthodontic appliance digital model based on the widths of the incisors in different dental regions, the widths between the canines, the widths between the premolars, and the widths between the molars;
Determining the overall length information of the orthodontic appliance digital model based on the positions of the molars in different dental regions.

4. The method according to claim 1, wherein determining the size information of the tooth sockets of the orthodontic model includes:
Determining the size information of the tooth sockets based on the labial-buccal and lingual contours of different dental arch regions.

5. The method according to claim 4, wherein the determination of the size information of the tooth groove includes:
Performing smoothing processing on the morphological data of the labial-buccal side contour and the lingual side contour, and determining the smoothed morphological data;
based on the smoothed morphological data, determining the size information of the tooth groove.

6. The method according to claim 1, further comprising following steps:
Based on the distance between the upper and lower occlusal surfaces of the different dental arch regions, the height of the jaw pad of the digital model of the orthodontic appliance is determined, the height of the jaw pad is used to generate the digital model of the orthodontic appliance.

7. The method according to claim 6, wherein the determination of the jaw pad height of the orthodontic appliance includes:
When the opening degree of the upper and lower jaws of the target oral digital model is less than the opening threshold, determine the distance between the upper occlusal surface and the lower occlusal surface.

8. The method according to claim 6, wherein the determination of the height of the jaw pad of the orthodontic appliance includes:
Determining the height of the jaw pad of the orthodontic appliance based on the resting intercuspation gap of different dental arch regions.

9. The method according to claim 1, further comprising following steps:
Based on the gum positions of the teeth in different dental arch regions, determine the outer wall length of the digital model of the orthodontic appliance, and the outer wall length is used to generate the digital model of the orthodontic appliance.

10. The method according to claim 9, wherein the determination of the outer wall length of the orthodontic appliance digital model includes:
Performing smoothing processing on the lines connecting the gum positions of different teeth in different dental regions;
based on the result of the smoothing processing, determining the outer wall length.

11. The method according to any one of claims 1 to 10, further comprising following step:
Based on the target oral parameters, adjust the patient's initial oral digital model to determine the target oral digital model.

12. The method according to claim 11, wherein the adjustment of the patient's initial oral digital model comprises:
Using the initial maxillary and mandibular digital models in the initial oral digital model, fitting a preset shape of the dental arch form to determine the target maxillary and mandibular digital models in the target oral digital model.

13. The method according to claim 11, wherein the adjustment of the patient's initial oral digital model comprises:
Adjusting the initial occlusal relationship in the initial oral digital model based on at least one of the preset overbite parameters, maxillary and mandibular dental arch parameters, overjet parameters, incisor relationship, molar relationship, or Spee curve, to determine the target occlusal relationship in the target oral digital model.

14. The method according to claim 11, wherein the initial oral digital model includes the initial upper and lower jaw digital models, the initial oral digital model includes at least one of the initial occlusal relationship or oral soft tissue information, the initial upper and lower jaw digital models are obtained through an intraoral scanner or the patient's dental impression, the initial occlusal relationship is obtained through an intraoral scanner or a jaw frame, and the oral soft tissue information is obtained through optical scanning technology, X-ray imaging technology, ultrasound imaging technology, computer tomography scanning or nuclear magnetic resonance technology.

15. The method according to one of claims 1 to 10, wherein the dental region comprises multiple areas such as the incisor area, the canine area, the premolar area or the molar area.

16. A device for generating a digital model of an orthodontic appliance comprises:
a determination unit, which is used to determine the overall size information and the size information of the dental arch of the orthodontic appliance digital model based on the relevant information of different tooth row areas of the target oral digital model;
a generation unit, which is used to generate the orthodontic appliance digital model based on the overall size information and the size information of the dental arch.

17. An electronic device, comprising:
an memory; and
a processor coupled to the memory, the processor being configured to execute the method for generating the digital model of the orthodontic appliance as claimed in any one of claims 1 to 15 based on the instructions stored in the memory.

18. A non-volatile computer-readable storage medium on which a computer program is stored, when executed by a processor, this program realizes the generation method of the digital model of the orthodontic appliance as claimed in any one of claims 1 to 15.

19. A manufacturing method for an orthodontic appliance comprises:
Using 3D printing technology to manufacture the orthodontic appliance based on the orthodontic appliance digital model generated according to the generation method of the orthodontic appliance digital model as described in any one of claims 1 to 15.

20. The manufacturing method according to claim 19, wherein,
use the first extrusion head to extrude the main material of the orthodontic appliance, and use the second extrusion head to extrude the supporting material.

21. The manufacturing method according to claim 20, wherein the first extrusion head and the second extrusion head can avoid each other during the printing process in accordance with the instructions.

22. The manufacturing method according to claim 20, wherein the printing of the main material for the orthodontic appliance using the first extrusion head and the printing of the supporting material using the second extrusion head include:
After the extrusion and stacking molding processing of all layers of the orthodontic appliance has been completed using the first extrusion head and the second extrusion head, the curing of all the main material layers is carried out.

23. The manufacturing method according to claim 20, wherein the main material is made from a curable silicone rubber formulation system.

24. The manufacturing method according to claim 23, wherein the thermosetting silicone rubber formulation contains vinyl silicone oil, hydrogen-containing silicone oil, platinum-based catalyst, and reinforcing agent.

25. The manufacturing method according to claim 24, wherein the reinforcing agent comprises silica.

26. The manufacturing method according to claim 23, wherein the thermosetting silicone rubber formulation contains components such as thixotropic agents.

27. The manufacturing method according to claim 20, wherein the hardness range of the main material is 40 to 80 Shore A.

28. The manufacturing method according to claim 20, wherein the hardness range of the main material is 50 to 70 Shore A.

29. The manufacturing method according to claim 20, wherein the hardness range of the main material is 55 to 65 Shore A.

30. The manufacturing method according to claim 20, wherein the tensile strength of the main material is greater than 3 MPa.

31. The manufacturing method according to claim 20, wherein the tensile strength of the main material is greater than 5 MPa.

32. The manufacturing method according to claim 20, wherein the elongation at break of the main material is greater than 70%.

33. The manufacturing method according to claim 20, wherein the elongation at break of the main material is greater than 100%.

34. The manufacturing method according to claim 20, wherein the tear strength of the main material is greater than 9 N/mm.

35. The manufacturing method according to claim 20, wherein the tear strength of the main material is greater than 10 N/mm.

36. The manufacturing method according to claim 20, wherein the diameter of the main material needle is 0.2 to 0.8 mm, the diameter of the main material needle is preferably 0.4 mm, the diameter of the supporting material needle is 0.2 to 0.8 mm, and the diameter of the supporting material needle is preferably 0.4 mm.

37. The manufacturing method according to claim 20, wherein the extrusion width of the main material is 0.2 to 0.8 mm, the extrusion width of the supporting material is 0.2 to 0.8 mm, the filling rate of the main material is 85 to 100%, the filling rate of the supporting material is 70 to 100%, the printing speed of the orthodontic appliance is 10 to 80 mm/s, the layer thickness is 0.2 to 0.3 mm, the extrusion width of the main material is preferably 0.4 mm, the extrusion width of the supporting material is preferably 0.4 mm, the filling rate of the main material is preferably 90%, the filling rate of the supporting material is preferably 90 to 100%, the printing speed of the orthodontic appliance is preferably 25 mm/s, and the layer thickness is preferably 0.2 mm.

38. A manufacturing device for an orthodontic appliance comprises:
An extrusion 3D printing system, which is used to manufacture the orthodontic appliance based on the generated digital model of the using orthodontic appliance 3D silicone printing technology, the digital model of the orthodontic appliance is generated through any one of the digital model generation methods described in claims 1 to 15.

39. An orthodontic appliance, the orthodontic appliance is manufactured by the manufacturing method of any one of claims 19 to 37.
